# EUROPEAN PATENT APPLICATION

(11) **EP 1 602 355 A1**
(43) Date of publication of application: **07.12.2005**
(21) Application number: 05253227.2
(22) Date of filing: 25.05.2005
(51) Int. Cl.: A61K 7/075, A61K 7/48, A61K 7/50

(54) **Cleansing foaming formulation**

(30) Priority: 25.05.2004 US 521565 P; 15.06.2004 US 710052
(71) Applicant: Coty Inc., New York, NY 10019 (US)
(72) Inventor: Knopf, Michael.A., Randolf, New Jersey 07869 (US); Polk, Michele., Flanders, New Jersey 07836 (US); Lucia, Frank.A., Wantage, New Jersey 07461 (US); Wohland, William.C., Succasunna, New Jersey 07876 (US); Macchio, Ralph., Sparta, New Jersey 07971 (US); Seplowitz, Paul. D., Orange, Connecticut 06477 (US)
(74) Representative: Campbell, Neil Boyd

(57) **Abstract**

A formulation comprising (I) a structurant; and (II) an emulsion comprising a homogenized mixture of wax and alcohol components; at least one of which comprises a surfactant, wherein the formulation comprises a stable lamellar or spherulite phase.

## Description

### Field of the Invention

Invention embodiments described herein relate to embodiments of personal care products that cleanse and moisturize skin of a user through foaming action, as well as a method of making the personal care product, and a method of using the personal care product.

### Background

Personal care products have a variety of applications, including topical application to skin. The topical applications have acted to moisturize, cleanse, disinfect or to apply active agents to the skin. Typically, the topical applications have performed only one of these functions. One type of personal care product, a product that cleanses skin has, in some instances, used a structurant in order to produce a composition having a lamellar or spherulite phase.

It has been reported that formation of lamellar dispersion based compositions can only be accomplished with a limited, small group of surfactants. Surfactants not falling within this small group have been reported to crystallize out of solution when added to a composition containing a structurant, or to destabilize the composition or both. The WO 97/05857 patent application includes a table that describes performances of a collection of test surfactants in forming a lamellar phase when added to a composition containing a structurant. The data in the table stated that stearyl alcohol, glyceryl monostearate and cetyl alcohol did not form lamellar phases at room temperature. Instead, these materials crystallized out of solution or destabilized the lamellar phase formed by the composition.

### Summary of the Invention

Viewed from one aspect the invention provides a formulation comprising (I) a structurant; and (II) an emulsion comprising a homogenized mixture of wax and alcohol components; at least one of which comprises a surfactant, wherein the formulation comprises a lamellar or spherulite phase.

Embodiments of the invention described herein include a formulation and system for cleansing and moisturizing skin, wherein the formulation and system include a stable, aqueous dispersion of cleansing and moisturizing agents that are structured within a stable, spheroidal network of finely divided cleansing and moisturizing particles. The stable, spheroidal network is capable of foaming due to flocculation in water and mechanical action by a consumer, which, in one embodiment, occurs in the shower. The spheroidal network also includes wetting agents and emulsifiers such as stearic acid, cetyl alcohol, glyceryl monostearate and stearyl alcohol, that are incorporated within the network. The wetting agents and emulsifiers are desirable because they aid in building viscosity of the formulation, and aid in producing a high yield value. Further, the wetting agents and emulsifiers aid in skin occlusiveness for increased moisturization. While specific types of wetting agents and emulsifiers are described herein, it is understood that the invention described herein is not limited to the specific wetting agents and emulsifiers described.

Embodiments of the invention additionally include methods for making the formulation and system, as well as methods for adding wetting agents and emulsifiers to a structurant in a manner that prevents the wetting agents and emulsifiers from "salting out." Furthermore, the wetting agents and emulsifiers such as stearic acid, cetyl alcohol, glyceryl monostearate and stearyl alcohol do not destabilize the activity of the structurant. Embodiments of the invention also include methods for using the formulation and system of the invention.

Prior to embodiments of the invention described herein, it has not been thought possible to incorporate wetting agents and emulsifiers such as stearic acid, cetyl alcohol, glyceryl monostearate, and stearyl alcohol into a formulation with a structurant to make a spheroidal network. Embodiments of the invention described herein include stearic acid, cetyl alcohol, glyceryl monostearate and stearyl alcohol as well as a structurant to make the spheroidal network. It has surprisingly been found that the stearic acid, cetyl alcohol, glyceryl monostearate, and stearyl alcohol do not salt out of the network and do not destabilize the network but are incorporated into the network without introducing instability. To the contrary, it has unexpectedly been found that these wetting agents and emulsifiers aid in building viscosity of the spheroidal network and aid in producing a high yield value. While stearic acid, cetyl alcohol, glyceryl monostearate, and stearyl alcohol are described, it is understood that other alcohols and waxes are suitable for use in the stable formulation of the invention.

As used herein, the term "lamellar" refers to an ordered liquid crystalline phase having alternating surfactant bilayers and water layers.

The term "spheroidal network" as used herein refers to a lamellar phase that conforms to form a submicron spherical onion. Vesicles and liposomes are types of spheroidal networks.

The term "structurant" as used herein refers to a molecule that aids in the formation of a spheroidal network.

The term "lotion" refers to a cosmetic formulation applied to the skin.

The term "wash" refers to a cosmetic formulation that is applied to the skin and then is washed from the skin.

The spheroidal network included in invention embodiments described herein has a multilayer structure conformed to a submicron onion shape. Insoluble materials are dispersed throughout the onion shaped spheroidal network. For some embodiments, insoluble materials are enclosed within the interior layers of the spheroidal network as well as the outer layers. Soluble materials are similarly dispersed throughout the spheroidal network, within interior layers and outer layers.

A structurant is a component of the formulation of the invention, e.g. a cleansing-moisturizing lotion of the invention. Compositions of the invention that employ structurants have lamellar or spherulitic phases that are, for example, capable of suspending large particles within the phase while remaining pourable. Structurants are also used to prepare product embodiments of the invention that impart a soft feel that is pleasing to consumers. Structurant may comprise an electrolyte-based structurant. Suitable electrolytes include an anion comprising phosphate, chloride, sulfate or citrate and a cation such as sodium, ammonium, potassium, or magnesium or mixtures thereof such sodium citrate. Examples of electrolyte-based structurants usable in the formulation and system embodiments of the invention described herein are described in WO01/05932, assigned to Huntsman and U.S. Patent Publication 20030190302, assigned to Rhodia. While specific electrolyte-based structurants are described herein, it is believed that other electrolyte-based structurants are suitable for use in embodiments of the invention.

The structurant preferably comprises a surfactant such as an anionic, a non-ionic, an amphoteric/zwitterionic or cationic surfactant. In particular, a mixture of surfactants can be employed, e.g. a mixture of anionic surfacatnst, anionic and catioinic surfactants or anionic and amphoteric/zwitterionic surfactants. Preferably, at least one of the surfactants has at least one unsaturated aliphatic group or a branched aliphatic group. Preferred anionic surfactants include an alkyl sulfate or alkyl ether sulfate. A salt of trideceth sulfate is a preferred anionic surfactant.

Preferred amphoteric/zwitterionic surfactants include amphoacetates, such as sodium lauroamphoacetate or cocoamphoacetate, or diamphoacetates. Preferred non-ionic surfactants include long chain (e.g. C8+) amido amine oxides, long chain amine oxides, e.g. cocoamide MEA.

One cleansing-moisturizing formulation of the invention has foaming functionality that aids in the cleansing functionality. This embodiment of the cleansing-moisturizing formulation includes at least five phases. Ingredients in a multi-phase formulation of the invention are shown in the table that follows. It is understood that this embodiment is presented as one example of the invention described herein and is not presented to limit the invention.

| **Phase A** | **% w/w** |
|---|---|
| Deionized Water | 34.880 |
| Guar hydroxypropyltrimonium chloride | 0.500 |
| EDTA disodium salt | 0.070 |

| **Phase B** | |
|---|---|
| Glycerine 99% USP | 1.000 |
| Cyamposis Tetragonoloba (Guar) Gum | 0.300 |
| Structurant Blend | 30.000 |

| **Phase C** | |
|---|---|
| Uvinul MS 40 Powder | 0.200 |
| | |

| **Phase D** | |
|---|---|
| Grape Seed Oil | 2.000 |
| Silicone 200/500 | 0.750 |
| Ethyl Hexyl Hydroxystearate | 1.000 |
| C12-15 Alkyl Ethyl Hexanoate | 0.750 |
| Stearic Acid Tri Press | 0.500 |
| Cetyl Alcohol | 0.500 |
| Glycerol Monostearate | 0.250 |
| Steryl Alcohol | 0.500 |
| Petrolatum | 6.000 |
| Shea Butter | 3.000 |
| Deionized Water | 10.000 |

| **Phase E** | |
|---|---|
| Fragrance | 1.500 |
| NaCl (Neat) | 5.000 |
| Citric Acid 20% Aq. Soln. | 1.000 |

| **Phase F** | |
|---|---|
| DMDM Hydantoin | 0.300 |

A first phase, phase A, of the formulation may include a deionized water diluent, a cationic conditioning agent, such as guar hydroxypropyltrimonium chloride, and a chelating agent, such as EDTA disodium salt or other non-toxic salts. Other cation conditioning polymers which are suitable for use in phase A, include Polyquatemium-4, Polyquatemium-6, Polyquaternium-7, Polyquaternium-10, Polyquaternium-11, Polyquaternium-16, Polyquarternium-24, and Polyquaternium-39. While specific quantities of ingredients are described in the table, it is understood that other concentration ranges may be suitable for use in formulation embodiments of the invention. The diluent range varies in accordance with other ingredients to reach a total concentration of 100 percent by weight. The cationic conditioning range may be from 0.001 to 1.000 percent by weight. The chelating agent range may be from 0.001 to 0.250 percent by weight.

Phase B may include glycerine, the structurant blend in a concentration of about 30 to 40 percent, and the Cyamopsis Tetragonoloba (Guar) Gum. In one embodiment, the structurant blend comprises Sodium Lauroamphoacetate, Sodium Trideceth Sulfate and Cocamide MEA.

Phase C, if present, may include Uvinul MS 40 Powder.

Phase D may include lotion ingredients such as grape seed oil, silicones, esters, wetting agents and emulsifiers such as stearic acid tri press, cetyl alcohol, glycerol monostearate, steryl alcohol, sodium lauryl sulphate, fatty alcohol, ether sulfates, disodium-n-lauryl-β-imino dipropionate, polyoxyethylinized castor oil, or sorbitan monooleate, sorbitan monostearate, lecithin, polyoxyethylene stearate, alkyl phenol polyglycol ether, cetyltrimethyl ammonium chloride, or mono-/dialkylpolyglycol ether-orthophosphorus acid- mono-ethanolamine salts, petrolatum and shea butter. Other ingredients are suitable for use in phase D to formulate a lotion.

Phase E may include sodium chloride and citric acid. The concentration range for use of sodium chloride may be from 1.000 to 6.000 percent by weight. The concentration range for use of citric acid may be from 0.001 to 3.000 percent by weight. If required, the pH is adjustable with sodium hydroxide or any other pH adjusting electrolyte, also within a range of 0.001 to 3.000 percent.

Phase F may include a preservative, such as DMDM hydantoin. Other preservatives suitable for use include phenoxyethanol, parabens, chlorophenesin, benzyl alcohol, chlorhexidine gluconate, an ethyl alcohol containing pentylene glycol and a sodium methylparaben mixture in the proportions 47/47/6, a pentylene glycol and sodium methylparaben mixture, Methylchloroisothiazolinone, Methylisothiazolinone, and mixtures thereof e.g. in a concentration range of 0.001 to 1.000 percent by weight, based upon proven efficacy per formula embodiment.

In addition to the above noted compounds, various other ingredients can optionally be utilized in the stable composition of the present invention such as Fragrances, Perfumes, Preservatives, Disinfectants, Deodorizers, Antiperspirants, Antioxidants, Antiredeposition Agents, Carriers, Chelating and Sequestering Agents, Dyes and Pigments, Quaternary Conditioners, Cationic conditioning polymers such as, Polyquatemium-4, Polyquaternium-6, Polyquaternium-7, Polyquaternium-10, Polyquaternium-11, Polyquaternium-16, Polyquartemium- 24, and Polyquaternium-39, Corrosion Inhibitors, Hydrotropes, Coupling Agents, Defoamers, Builders, Dispersants, Emollients, Extracts, Vitamins, Enzymes, Foam Boosters, Flocculants, Whitening Agents, Fixative Polymers such as PVP, Humectants, Opacifiers, Plasticizers, Powders, Solubilizers, Solvents, Waxes, UV Absorbers/UV Light Stabilizers, Hydrolyzed Proteins, Keratin, Collagens, and the like.

In the formulation embodiment of the table described above, Phase A was prepared by mixing the guar hydroxypropyltrimonium chloride and deionized water until the guar hydroxypropyltrimonium chloride was thoroughly blended to form a uniform phase A mixture. The EDTA disodium salt was then added to the phase A mixture.

To make phase B, the Cyamopsis Tetragonoloba (Guar) Gum and glycerine are blended until the Cyamopsis Tetragonoloba (Guar) Gum was wetted and was completely dispersed to make a slurry. The glycerine/Cyamopsis Tetragonoloba (Guar) Gum slurry was added to the structurant blend and was mixed until a uniform blend was achieved. The phase B uniform blend was then added to the phase A mixture to form a combined phase A and B mixture.

The combined phase A and B mixture is mixed until uniform. The uniform mixture is then heated to a temperature within a range of 40 degrees C. to 70 degrees C. Phase C was added to the combined phase A and B mixture to make a phase A, B and C mixture.

To make phase D, the following ingredients were blended: Grape seed oil, silicone 200/500, EthylHexyl Hydorstearate, C12-15 Alkyl EthylHexanoate, stearic acid tri pres, cetyl alcohol, glycerol monostearate, stearyl alcohol, petrolatum, shea butter and deionized water. The phase D mixture was heated until all ingredients were liquid. When all ingredients were liquid, rendering phase D liquid, phase D was high energy mixed at moderate speed. The moderate speed mixing was continued until the mixture was ready to add to the combined mixture of phases A, B and C, also called the main batch.

Phase D was then added to make a combined blend of phase A, B, C and D. The mixture of phase A, phase B, phase C and phase D was mixed until a homogeneous mixture was achieved. The mixture was cooled to room temperature at a rate of 1 degree Centigrade for every 10 minutes. The ingredients of phases E and F were added, one at a time, until a homogeneous mixture that had all of the desired attributes of the cleansing foaming lotion embodiment of the invention were achieved.

The cleansing foaming lotion embodiments of the invention described herein may be viscous liquids, having a viscosity of about 100,000 cPs and a pH of 5.5 to 6.5. The wash embodiments, are, for some embodiments, colored or fragranced. Some embodiments of the cleansing foaming lotion may moisturize skin for at least about 24 hours from application.

One lotion embodiment of the invention foams and cleanses for use in the shower and may moisturize skin for at least about 24 hours after showering. With this embodiment, a user need use only a product of the invention to both cleanse and moisturize. A separate cleanser and moisturizer are not required. That the invention described herein is capable of both cleansing and moisturizing is unexpected because cleansing skin removes fats, oils and lipids from the skin. Cleansing typically leaves skin dehydrated. It is then unexpected that a single product is capable of both cleansing and moisturizing.

One additional attribute of the lotion of the invention described herein is that the cleansing moisturizing lotion moisturizes skin without leaving a heavy oil feel on the skin. This heavy moisturized feel typically occurs when a moisturizer is separately applied to skin. The lotion described herein not only saves a user time and money in not having to apply two separate products but eliminates the heavy oil feel while effectively moisturizing.

The cleansing foaming lotion of the invention described herein differs from a traditional wash in that the traditional wash does not include a spherulite state. This spherulite state is also known as an "onion phase" state. The spherulite state is a stable high energy state. The spherulites within the formulation make it possible to add lotion ingredients to a cleansing and foaming product and to maintain all of the properties of both the body wash, which cleanses and the lotion, which moisturizes.

The cleansing foaming lotion is a structured liquid formulation that includes water soluble, water dispersible, water insoluble and water indispersible ingredients without an adverse impact, such as "salting out" and incipient instability. The cleansing portion of the formulation also includes adjuvants and solubilizers that aid in creating a product with a pre-selected viscosity or foaming potential. The formulation of the invention described herein produces a finished product that has improved stability as compared to conventional washes because of materials in the formulation that add stability, and that cannot be added to a conventional wash. These materials include wetting agents and emulsifiers such as stearic acid, cetyl alcohol, glyceryl monostearate and stearyl alcohol.

A method for making a formulation embodiment of the invention that includes waxes and alcohols such as stearic acid, cetyl alcohol, glyceryl monostearate and stearyl alcohol, or other wax or alcohol-based surfactant, includes the steps described herein. One embodiment of Phase D, such as is described above, was prepared by adding oils, silicones, esters and any other insoluble ingredients to the alcohols and waxes. About 10 percent deionized water was also added.

Phase D was heated to about 70 degrees Centigrade to melt the alcohols and waxes. When all of the alcohols and waxes were melted, phase D was emulsified and homogenized using, in one embodiment, a homo-mixer at moderate speed. Phase D was mixed for a minimum of five minutes. Moderate sidesweep mixing was continued until the phase D was ready for addition to the phase A, B and C mixture. Phase D was then added to the phase A, Band C mixture when the A, B, and C mixture was mixed homogeneously. An addition of sodium chloride and/or citric acid and /or fragrance drove the formula to a spherulite phase. It was observed that the spherulite phase was stable with no salting out.

One test for determining whether the formulation is in a spherulite phase includes taking a 100 gram formulation sample of the formulation and adding 1 to 2 percent neat salt. If, at 24 hours the viscosity has increased above the initial viscosity of the batch, the formulation is not in the spherulite phase. This result indicates that the formulation requires more oils or salt in order to form a spherulite phase. A second test includes preparing a sample of about 100 grams and adding several beads to the sample. The sample is held at a temperature of about 50 degrees Centigrade for several days. If the beads are still suspended, the sample has a spherulite phase and is stable.

Embodiments of the formulation of the invention described herein have use in shaving and moisturizing, shampoos and conditioners, in addition to washes and moisturizers. Formulation embodiments of the invention described herein have a wide number of other applications such as personal care applications, home care applications, industrial and institutional applications, pharmaceutical applications, textile compounds, and the like.

Examples of various personal care applications include products such as the following: Shampoos, for example Baby Shampoos; Conditioning Shampoos; Bodifying Shampoos; Moisturizing Shampoos; Temporary Hair Color Shampoos; 3-in-1 Shampoos; Anti-Dandruff Shampoos; Hair Color Maintenance Shampoos; Acid (Neutralizing) Shampoos; Salicylic Acid Shampoos;

Skin and Body Cleansers, for example Moisturizing Body Washes; Antibacterial Body Washes; Deodorizing Body Washes; Bath Gels; Shower Gels; Hand Soaps; Bar Soaps; Body Scrubs; Bubble Baths; Facial Scrubs; Foot Scrubs; Creams and Lotions, for example Alpha-Hydroxy Acid Lotions and Creams; Beta-Hydroxy Acid Creams and Lotions; Skin Whiteners; Self Tanning Lotions; Sunscreen Lotions; Barrier Lotions; Moisturizers; Hair Styling Creams; Vitamin C Creams; Liquid Talc Products and Antibacterial Lotions; and other moisturizing lotions and creams;

Skin and Hair Gels, for example Facial Masks; Body Masks; Hydroalcoholic Gels; Hair Gels; Body Gels; Sunscreen Gels; and the like, as well as other personal care applications such as permanent hair color, and the like.

Examples of home care applications include products such as home care and industrial and institutional applications, such as laundry detergents; dishwashing detergents (automatic and manual); hard surface cleaners; hand soaps, cleaners and sanitizers; polishes (shoe, furniture, metal, etc.); automotive waxes, polishes, protectants, and cleaners, and the like.

Examples of pharmaceutical applications include topical formulations in the form of creams, lotions, ointments, or gels, wherein the formulation may be used as a carrier for the pharmaceutically active material, or as a carrier for a skin penetration enhancer, or as a carrier for a phase having an aesthetic effect, or present to enhance the solubility or bioavailability of the pharmaceutically active material.

These formulations may be administered or applied to either human or veterinary conditions for the full breadth of indications treatable by pharmaceutical means, such as fever, irritation, dermatitis, rash; viral, fungal, or bacterial infection; organic disease; etc. The pharmaceutically active agents could have any appropriate function for treatment of the condition, and can be a mixture of one or more pharmaceutically active materials, such as emetics, antiemetics, febrifuge, fungicide, biocide, bactericide, antibiotic, antipyretic, NSAID, emollient, analgesics, antineoplastics, cardiovascular agents, enzymes, proteins, hormones, steroids, antipruritics, antirheumatic agents, biologicals, cough and cold treatments, dandruff products, muscle relaxants, psychotherapeutic agents, skin and mucous membrane agents, skin care products, vaginal preparations, wound care agents, and other appropriate classes of pharmaceutically active agents capable of appropriate administration via dosage form.

The formulation embodiments may be packaged in a pressurized container or unpressurized container. The formulation may be applied to wipes, swabs or other flexible substrates.

The formulation embodiments may include variegation, and suspended solids that impart color. The formulation embodiments may be made into a wide variety of product types that include, but are not limited to, lotions, creams, gels, sticks, sprays, ointments, cleansing liquid washes, solid bars, shampoos, pastes, foams, powders, mousses, shaving creams, wipes, patches, nail lacquers, wound dressing, adhesive bandages, hydrogels, and films. Make-up, such as foundations, mascaras, and lipsticks also form suitable compositions. These product embodiments may further comprise several additional types of cosmetically acceptable topical carriers including, but not limited to solutions, emulsions (e.g., microemulsions and nanoemulsions), gels, solids and liposomes.

While certain embodiments of the present invention have been described and specifically exemplified above, it is not intended that the invention be limited to such embodiments. Various modifications may be made thereto without departing from the scope and spirit of the present invention, as set forth in the following claims.

## Claims

1. A formulation comprising (I) a structurant; and (II) an emulsion comprising a homogenized mixture of wax and alcohol components; at least one of which comprises a surfactant,
wherein the formulation comprises a stable lamellar or spherulite phase.

2. The formulation of claim 1 wherein the wax and alcohol components are melted.

3. The formulation of claim 1 or 2 wherein the structurant comprises an electrolyte.

4. The formulation of claim 1 to 3, wherein the emulsion further comprises grape seed oil.

5. The formulation of claim 1 to 4, wherein the emulsion further comprises one or more silicone.

6. The formulation of claim 1 to 5, wherein the emulsion further comprises one or more ester.

7. The formulation of claim 1 to 6, wherein the emulsion further comprises water.

8. The formulation of claim 1 to 7, wherein the wax and alcohol components comprise one or more of stearic acid, cetyl alcohol, glycerol monostearate, and stearyl alcohol.

9. The formulation of claim 1 to 8, further comprising water.

10. The formulation of claim 1 to 9, further comprising a fragrance.

11. The formulation of claim 1 to 10 further comprising one or more ingredients that moisturize skin.

12. The formulation of claim 11 wherein the ingredients that moisturize skin comprise one or more of grape seed oil, silicone, and esters.

13. The formulation of claim 1 to 12 further comprising one or more ingredients that cleanse skin.

14. The formulation of claim 1 to 13, further comprising one or more ingredients that cleanse and moisturize skin.

15. The formulation of claim 1 to 14 comprising at least five phases.

16. The formulation of claim 15, comprising a lotion phase.

17. The formulation of claim 16, wherein the lotion phase comprises one or more of stearic acid, cetyl alcohol, glycerol monostearate, and steryl alcohol.

18. An after-shave, shower wash, shampoo, wipe, swab, pharmaceutical, variegated cosmetic or skin care product comprising the formulation of claim 1 to 17.

19. A method for making a formulation with a stable lamellar or spherulite phase comprising: preparing a phase comprising a structurant; preparing a mixture comprising at least one alcohol and at least one wax; heating the mixture to melt the alcohol or alcohols and wax or waxes to form a melted mixture; homogenizing the melted mixture and adding the structurant phase to the homogenized phase.

20. The method of claim 19, further comprising adding one or more of oils, silicones, esters, and other water insoluble ingredients to the melted mixture before homogenizing the phase.

21. The method of claim 19 or 20, further comprising adding a salt or acid or fragrance to create a lamellar or spherulite phase.

22. The method of claim 19 to 21, further comprising adding additional phases to make a lotion or wash.

23. The method of claim 19 to 22, further comprising adding water to the melted mixture.
